(19) Europäisches Patentamt
European Patent Office
Office européen des brevets

(11) Veröffentlichungsnummer: **0 209 738**
**A1**

(12) # EUROPÄISCHE PATENTANMELDUNG

(21) Anmeldenummer: 86108466.3

(22) Anmeldetag: 20.06.86

(51) Int. Cl.⁴: C07D 249/08 , A01N 43/64

(30) Priorität: 15.07.85 DE 3525221

(43) Veröffentlichungstag der Anmeldung: 28.01.87 Patentblatt 87/05

(84) Benannte Vertragsstaaten: AT BE CH DE FR GB IT LI LU NL SE

(71) Anmelder: **CHEMIE LINZ AKTIENGESELLSCHAFT**
St. Peter-Strasse 25
A-4020 Linz(AT)
Anmelder: **Lentia Gesellschaft mit beschränkter Haftung**
Arabellastrasse 4 Postfach 81 05 08
D-8000 München 81(DE)

(72) Erfinder: **Saischek, Gerald, Dipl.-Ing.**
Rosesggerstrasse 4
A-4600 Wels(AT)
Erfinder: **Kirchner, Gerald, Dipl.-Ing.Dr.**
Boschweg 2a
A-4020 Linz(AT)
Erfinder: **Fuchs, Franz**
Schumpeterstrasse 2
A-4040 Linz(AT)
Erfinder: **Schneider, Rudolf, Dipl.-Ing.Dr.**
Prandtauerstrasse 63
A-4040 Linz(AT)
Erfinder: **Bodingbauer, Robert, Mag.Dr.**
Eben 72
A-4202 Hellmonsödt(AT)
Erfinder: **Graf, Josef**
Kürnberg 116
A-4442 Kleinraming(AT)

(54) **Benzoyl-triazolyl-essigsäure- oder -thioessigsäurederivate, verfahren zu deren Herstellung und diese enthaltende biozide Mittel.**

(57) Benzoyl-triazolyl-essigsäure-oder thioessigsäurederivate der allgemeinen Formel I des Formelblattes,

in der $R_1$ und $R_2$ unabhängig voneinander Wasserstoff, Halogen oder einen Alkoxyrest mit 1 -4 C-Atomen, Y Sauerstoff oder Schwefel, Z die Reste $OR_4$, $SR_4$ oder $R_5NR_6$, wobei $R_4$ einen geradkettigen oder verzweigten, gesättigten oder ungesättigten, unsubstituierten oder ein-oder mehrfach durch Halogen-, Alkoxy-oder Alkoxycarbonyl-mit 1 bis 4 C-Atomen in der Alkylkette substituierten Kohlenwasserstoffrest mit 1 bis 20 C-Atomen oder einen cyclischen Kohlenwasserstoffrest, der durch Alkyl-oder Alkoxycarbonylreste mit 1 -4 C-Atomen in der Alkylkette substituiert sein kann und $R_5$ und $R_6$ unabhängig voneinander einen Alkyl-, einen Alkoxycarbonylrest mit 1 -4 C-Atomen in der Alkylkette, einen Phenylrest oder $R_5$ und $R_6$ gemeinsam mit dem Stickstoffatom einen Morpholinoring, der durch Alkylreste mit 1 -4 C-Atomen substituiert sein kann, bedeuten und deren pflanzenphysiologisch verträgliche Salze und Metallkomplexe, Verfahren zu deren Herstellung sowie diese enthaltende biozide Mittel, Verfahren zu deren Herstellung und deren Verwendung.

EP 0 209 738 A1

Xerox Copy Centre

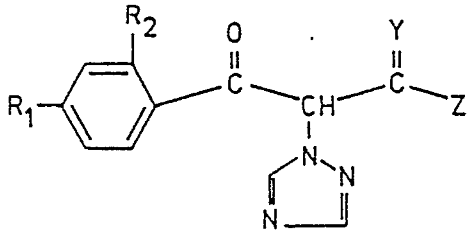

I

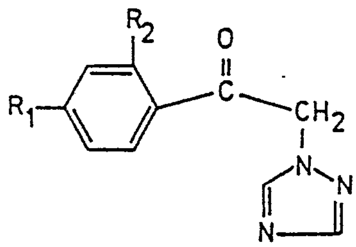

II

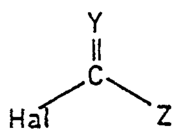

III

# Benzoyl-triazolyl-essigsäure-oder -thioessigsäurederivate, deren Herstellung und diese enthaltende biozide Mittel

Die Erfindung betrifft neue Benzoyl-triazolyl-essigsäure-oder -thioessigsäurederivate, Verfahren zu deren Herstellung sowie diese enthaltende biozide Mittel, Verfahren zu deren Herstellung und deren Verwendung.

In der EP-A 008458 und in der DE-OS 28 31 235 sind Azolylmethylmalonsäurederivate beschrieben, die als Schädlingsbekämpfungsmittel bzw. nematizide Mittel eingesetzt werden können. Die Wirkung und die Wirkungsdauer dieser Verbindungen ist jedoch, insbesondere bei bestimmten Schadorganismen nicht immer völlig zufriedenstellend.

Unerwarteterweise wurden neue Benzoyl-triazolyl-essigsäure-oder -thioessigsäurederivate gefunden, die ausgezeichnete biozide Eigenschaften aufweisen.

Gegenstand der Erfindung sind demnach Benzoyltriazolyl-essigsäure-oder -thioessigsäurederivate der allgemeinen Formel I des Formelblattes,

in der $R_1$ und $R_2$ unabhängig voneinander Wasserstoff, Halogen oder einen Alkoxyrest mit 1 -4 C-Atomen, Y Sauerstoff oder Schwefel, Z die Reste $OR_4$, $SR_4$ oder $R_5NR_6$, wobei $R_4$ einen geradkettigen oder verzweigten, gesättigten oder ungesättigten, unsubstituierten oder ein-oder mehrfach durch Halogen-, Alkoxy-oder Alkoxycarbonyl-mit 1 bis 4 C-Atomen in der Alkylkette, Phenyl-, Halogenphenyl oder Trialkylsilylreste mit 1 -4 C-Atomen in der Alkylkette substituierten Kohlenwasserstoffrest mit 1 bis 20 C-Atomen oder einen cyclischen Kohlenwasserstoffrest, der durch Alkyl-oder Alkoxycarbonylreste mit 1 -4 C-Atomen in der Alkylkette substituiert sein kann und $R_5$ und $R_6$ unabhängig voneinander einen Alkyl-, einen Alkoxycarbonylrest mit 1 -4 C-Atomen in der Alkylkette, einen Phenylrest oder $R_5$ und $R_6$ gemeinsam mit dem Stickstoffatom einen Morpholinoring, der durch Alkylreste mit 1 -4 C-Atomen substituiert sein kann, bedeuten und deren pflanzenphysiologisch verträgliche Salze und Metallkomplexe.

Weiters wurde gefunden, daß man die erfindungsgemäßen Substanzen erhält, indem man ein Keton der allgemeinen Formel II des Formelblattes,

in der $R_1$ und $R_2$ die in Anspruch 1 angegebene Bedeutung haben, mit einer Verbindung der allgemeinen Formel III des Formelblattes,

in der Y und Z die in Anspruch 1 angegebene Bedeutung haben und Hal Chlor oder Brom bedeutet, in Gegenwart der mindestens äquivalenten Menge eines Säureakzeptors und gegebenenfalls in Gegenwart eines unter Reaktionsbedingungen inerten Verdünnungsmittels bei einer Temperatur von -20°C bis +140°C umsetzt, gegebenenfalls das Verdünnungsmittel entfernt und das Reaktionsprodukt gegebenenfalls in ein pflanzenphysiologisch verträgliches Salz oder Metallkomplex überführt.

Die erfindungsgemäßen Benzoyl-triazolyl-essigsäure-und -thioessigsäurederivate zeigen ausgezeichnete biozide Eigenschaften und stellen somit eine Bereicherung der Technik dar.

In der Formel I bedeuten $R_1$ und $R_2$ unabhängig voneinander ein Wasserstoffatom, ein Halogenatom oder einen Alkoxyrest mit 1 bis 4 C-Atomen, vorzugsweise ein Halogenatom oder den Methoxyrest, besonders bevorzugt ein Chloratom.

Y bedeutet Sauerstoff oder Schwefel, besonders bevorzugt Sauerstoff.

Z kann die Reste $OR_4$, $SR_4$ oder $R_5NR_6$ bedeuten, wobei die Reste $OR_4$ und $SR_4$ besonders bevorzugt sind.

$R_4$ bedeutet dabei einen geradkettigen oder verzweigten, gesättigten oder ungesättigten Kohlenwasserstoffrest mit 1 -20 C-Atomen. Beispiele für solche Reste sind gesättigte Kohlenwasserstoffreste, wie Methyl-, Ethyl-, n-Propyl, -i-Propyl-, Butyl-, sec.-Butyl-, t-Butylreste, geradkettige oder verzweigte Pentyl-, Hexyl-, Heptyl-, Octyl-, Dodecyl-, Tridecyl-, Hexadecyl-, Nonadecylreste, ethylenisch ungesättigte, geradkettige oder verzweigte Kohlenwasserstoffreste, wie Vinyl-, Allyl-, Propenyl-, i-Propenyl-, Butenyl-, Pentenylreste, acetylenisch ungesättigte Kohlenwasserstoffreste, wie Ethinyl-, Propinyl-, 1-Butinyl-, 2-Butinyl-oder Heptinylreste. Bevorzugt sind jene Verbindungen, in denen $R_4$ einen gesättigten Alkylrest mit 1 bis 18 C-Atomen oder einen ethylenisch oder acetylenisch ungesättigten Kohlenwasserstoffrest mit 1 bis 8 C-Atomen bedeutet.

Ferner kann der Kohlenwasserstoffrest ein-oder mehrfach durch Halogenatome, Alkoxy-oder Alkoxycarbonylreste mit 1 -4 C-Atomen in den Alkylketten, oder durch gegebenenfalls ein-oder mehrfach halogenierte Phenylreste oder durch einen Trialkylsilylrest mit 1 bis 4 C-Atomen in den Alkylketten substituiert sein. Beispiele für solche substituierten Kohlenwasserstoffreste sind ein oder mehrfach halogenierte Alkylreste, wie 1-Chlorethyl-, 1-Chlorpropyl-, 1-Bromethyl-, 2-Brompropyl-, 1-Brompropyl-, Dichlormethyl-, 1,1-Dichlorethyl-, 1,1,1-Trichlorethyl-und die verschiedenen Trichlorbutylreste, weiters durch Alkoxy oder Alkoxycarbonyl substituierte Alkylreste, wie Methoxyethyl-,

Methoxypropyl-, Methoxybutyl-, Ethoxypropyl-, Ethoxyheptyl-, Propoxyhexyl-, Methoxycarbonylpropyl-, Ethoxycarbonylbutyl-, Propoxycarbonylbutylreste. Beispiele für durch gegebenenfalls halogenierte Phenylreste substituierte Alkylreste sind Benzyl-, 4-Butylphenyl-, 4-Ethylphenyl-, Monochlorbenzyl-, Dichlorbenzyl-, Brombenzylreste, Beispiele für durch Trialkylsilylreste substituierte Alkylreste sind Trimethylsilylpropyl-, Triethylsilylethyl-oder Tripropylsilylhexylreste.

Bevorzugt sind jene Verbindungen, in denen $R_4$ einen durch Halogenatome ein-oder mehrfach substituierten Alkylrest mit 1 -18 C-Atomen, einen durch Alkoxyreste mit 1 -4 C-Atomen oder gegebenenfalls halogeniertes Phenyl substituierten Alkylrest mit 1 -18 C-Atomen bedeutet.

Besonders bevorzugt sind jene Verbindungen, in denen $R_4$ einen gegebenenfalls ein-oder mehrfach durch Chloratome substituierten Alkylrest mit 1 -15 C-Atomen oder den Benzyl-oder den 2,4-Dichlorbenzylrest bedeutet.

Weiters kann $R_4$ einen cyclischen Kohlenwasserstoffrest, wie z.B. einen Cyclohexylrest, einen Phenylrest, einen durch Alkylreste substituierten, cyclischen Kohlenwasserstoffrest, wie den 4-Methylcyclohexyl-, den 4-Ethylcyclohexyl-oder den 4-Butylcyclohexylrest oder einen durch Alkylcarbonyl substituierten, cyclischen Kohlenwasserstoffrest, wie den Methylcarbonylphenylrest oder den Ethylcarbonylphenylrest, bedeuten.

Bevorzugt sind solche Verbindungen, in denen $R_4$ einen Cyclohexylrest bedeutet.

$R_5$ und $R_6$ können gleich oder verschieden sein und je einen Alkylrest mit 1 bis 4 C-Atomen, wie den Methyl-, Ethyl-, Propyl-oder Butylrest, einen Alkoxycarbonylrest mit 1 bis 4 C-Atomen in der Alkylkette, wie z.B. den Methoxycarbonyl-, den Ethoxycarbonyl-oder den Propoxycarbonylrest bedeuten.

Bevorzugt sind jene Verbindungen, in denen $R_5$ und $R_6$ je einen Alkylrest mit 1 -4 C-Atomen bedeutet.

$R_5$ und $R_6$ können weiters gemeinsam mit dem Stickstoffatom einen gegebenenfalls ein-oder mehrfach durch Alkylreste mit 1 -4 C-Atomen substituierten Morpholinoring bilden, wie den 3,5-Dimethylmorpholinorest.

Die erfindungsgemäßen Verbindungen können hergestellt werden, indem man ein Keton der allgemeinen Formel II mit der äquimolaren Menge einer Verbindung der allgemeinen Formel III in Gegenwart von mindestens der äquivalenten Menge eines Säureakzeptors umsetzt.

Dabei wird üblicherweise zuerst mit dem Säureakzeptor ein Salz des Ketons der allgemeinen Formel II hergestellt und dieses Salz anschließend mit einer Verbindung der allgemeinen Formel III umgesetzt. Es ist aber auch möglich, die erfindungsgemäßen Verbindungen in einem Schritt durch gleichzeitigen Einsatz der Reaktionspartner und des Säureakzeptors herzustellen.

Als Säureakzeptoren dienen stark basische Verbindungen, wie z.B. anorganische Basen, wie Natriumhydrid oder Kaliumhydrid, oder organische Basen, wie z.B. Trialkylaminverbindungen, wie Trimethylamin, Triethylamin, Tripropylamin oder Triphenylaminverbindungen.

Vorteilhafterweise erfolgt die Umsetzung in einem unter Reaktionsbedingungen inerten Verdünnungsmittel, etwa in halogenierten oder nichthalogenierten Kohlenwasserstoffen, wie Petroläther, Toluol, Chloroform, Methylenchlorid oder in etherartigen Verdünnungsmitteln, wie Diethylether, Dioxan oder Tetrahydrofuran.

Die Umsetzung kann weiters mit einem Überschuß des flüssigen Säureakzeptors erfolgen, der in diesem Fall zugleich als Lösungs-oder Verdünnungsmittel dient.

Die Reaktion wird bei Temperaturen von etwa -20°C bis +140°C durchgeführt, vorzugsweise bei Temperaturen von -10°C bis +80°C, besonders bevorzugt bei Temperaturen von 0°C bis 35°C.

Die Reaktionsdauer beträgt je nach Reaktivität der Ausgangsverbindungen, Aktivität des Säureakzeptores und Reaktionstemperatur etwa 30 Minuten bis 5 Stunden, üblicherweise etwa 1 bis 2 Stunden.

Nach beendeter Reaktion wird das entstandene anorganische oder organische Salz durch Waschen mit Wasser oder Abfiltrieren entfernt. Anschließend werden die flüchtigen Bestandteile des Reaktionsgemisches bei Atmosphärendruck oder vermindertem Druck bei Raumtemperatur oder erhöhter Temperatur verdampft.

Gegebenenfalls kann das verbleibende Produkt durch geeignete Maßnahmen, wie z.B. Umkristallisieren, Extraktion oder chromatographische Trennung weiter gereinigt werden.

Die als Ausgangssubstanzen verwendeten Verbindungen der allgemeinen Formeln II und III sind bekannt und können nach an sich bekannten Methoden hergestellt werden. So können Verbindungen der allgemeinen Formel II beispielsweise nach der DE-OS 26 53 420 hergestellt werden.

Zur Herstellung der Salze oder Metallkomplexe wird die freie Base der allgemeinen Formel I in Gegenwart eines inerten Verdünnungsmittels mit mindestens der stöchiometrischen Menge einer entsprechenden Säure oder eines entsprechenden Metallsalzes umgesetzt. Vorteilhafterweise werden die einzelnen Reaktanten in Lösung eingesetzt.

Der dabei entstehende Niederschlag wird etwa durch Filtrieren, gegebenenfalls unter Kühlung abgetrennt, gewaschen und anschließend getrocknet.

Die Produkte fallen üblicherweise in großer Reinheit an und können gegebenenfalls durch Umkristallisieren weiter gereinigt werden.

Die erfindungsgemäßen Verbindungen und ihre Salze und Metallkomplexverbindungen sind gegen ein breites Spektrum von pflanzenpathogenen Pilzen, Algen und Bakterien wirksam, z.B. gegen Pilze aus den Klassen der Phycomyceten, Ascomyceten, Basidiomyceten und Deuteromyceten.

Die gute Pflanzenverträglichkeit und die z.T. systemische Wirksamkeit in den zur Behandlung von Pflanzenkrankheiten notwendigen Konzentrationen erlauben eine Behandlung von oberirdischen Pflanzenteilen, von Pflanz-und Saatgut und des Bodens.

Besonders interessant sind die fungiziden Verbindungen für die Bekämpfung einer Vielzahl von Pilzen an verschiedenen Kulturpflanzen und ihren Samen, wie z.B. Weizen, Roggen, Gerste, Hafer, Reis, Mais, Baumwolle, Soja, Kaffee, Zuckerrohr, Obst und Zierpflanzen im Gartenbau sowie in Gemüsen wie Gurken, Bohnen oder Kürbisgewächsen.

Diese neuen Verbindungen können mit besonders gutem Erfolg etwa zur Bekämpfung folgender Pflanzenkrankheiten eingesetzt werden:

| | |
|---|---|
| Erysiphe graminis | an Getreide |
| Erysiphe cichoracearum | an Kürbisgewächsen |
| Erysiphe betae | an Zuckerrüben |
| Puccinia-Arten | an Getreide |
| Uromyces phaseoli | an Bohnen |
| Ustilago-Arten | an Getreide |
| Venturia inaequalis | an Äpfeln |
| Podosphaera leucotricha | an Äpfeln |
| Septoria nodorum | an Weizen |
| Septoria apii | an Sellerie |
| Alternaria solani | an Kartoffeln |
| Helminthosporium avenae | an Gerste |
| Pseudocercosporella herpotrichoides | an Getreide |
| Tilletia caries | an Weizen |
| Rhizoctonia solani | an Kartoffeln und Baumwolle |
| Marssonina rosae | an Rosen. |

In entsprechenden Aufwandmengen können die Stoffe auch als Holzschutzmittel gegen holzverfärbende Pilze und Moderfäulepilze eingesetzt werden, wie z.B. zur Bekämpfung von Merulius lacrimans, Polyporus vaporarius, Poria placenta und Polystictus cinnabarinus.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3,00 kg Wirkstoff oder mehr je ha.

Die Wirkstoffe können je nach ihrem Anwendungsgebiet in die üblichen Formulierungen übergeführt werden, wie Lösungen, Spritzpulver, Emulsionskonzentrate, Emulsionen, Suspensionen, Pulver, Schäume, Pasten, Granulate, Aerosole, wirkstoffimprägnierte Natur-und synthetische Stoffe, Feinstverkapselungen in polymeren Stoffen und

in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden, verflüssigten Gasen und/oder festen Trägerstoffen, gegebenenfalls unter Verwendung von Tensiden, also Emulgatoren und/oder Dispergier-und/oder Netzmitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten, gasför migen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe, sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Produkte, wie hochdisperse Kieselsäure, Aluminiumoxyd und Silikate; als feste Trägerstoffe für Granulate kommen in Frage: z.B. gebrochene und fraktionierte natürliche Gesteine, wie Calcit, Marmor, Bims, Sepiolith, Dolomit sowie synthetische Granulate aus anorganischen und organischen Mehlen, sowie Granulate aus organischem Material, wie Sägemehl, Kokosnußschalen, Maiskolben und Tabakstengel; als Emulgiermittel und/oder schaumerzeugende Mittel kommen in Frage: z.B. nichtionogene und ionogene Tenside, wie Polyoxyethylen-Fettsäure-Ester, Polyoxyethylen-Fettalkoholether, z.B. Alkylarylpolyglykolether, Alkylsulfonate, Alkylsulfate, Arylsulfonate und Arylalkylsulfonate sowie Eiweißhydrolysate; als Dispergiermittel kommen in Frage z.B. Ligninsulfonate oder Kondensationsprodukte von Arylsulfonaten mit Formaldehyd.

Es können in den Formulierungen Haft-und Verdickungsmittel, wie Carboxymethylcellulose, Methylcellulose, natürliche und synthetische pulverige, körnige und latexförmige Polymere, wie Gummiarabicum, Polyvinylalkohol, Polyvinylacetat verwendet und anorganische oder organische Farbstoffe zugesetzt werden.

Die Formulierungen enthalten im allgemeinen zwischen 0,1 und 95 Gew.% Wirkstoff, vorzugsweise zwischen 0,5 und 90 Gew.%.

Die Wirkstoffe können als solche in Form ihrer Formulierungen oder den daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.%, vorzugsweise zwischen 0,5 und 0,001 Gew.%.

Bei der Saatgutbehandlung werden im allgemeinen Wirkstoffmengen von 0,001 bis 50 g je Kilogramm Saatgut, vorzugsweise 0,01 bis 10 g, benötigt.

Bei der Behandlung des Bodens sind Wirkstoffkonzentrationen von 0,00001 bis 0,1 Gew.%, vorzugsweise von 0,0001 bis 0,02 Gew.%, am Wirkungsort erforderlich.

Beispiel 1:

In einem Reaktionskolben wurden 14,08 g -(0,055 mol) 2-(2,4-Dichlorphenyl)-1-(1,2,4-triazol-1-yl)-ethan-2-on in 150 ml Tetrahydrofuran gelöst und 1,32 g (0,055 mol) Natriumhydrid zugesetzt. Nach zweistündigem Rühren bei Raumtemperatur wurden 6,0 g (0,055 mol) Chlorameisensäureethylester in 50 ml Tetrahydrofuran zugetropft. Nach einer Stunde wurde das Lösungsmittel bei Raumtemperatur unter vermindertem Druck entfernt, der Rückstand in Chloroform aufgenommen, mit Wasser gewaschen, die organische Phase getrocknet und das Lösungsmittel verdampft. Der ölige Rückstand wurde in Tetrachlormethan/Acetonitril (v/v : 3/1) gelöst und mittels Säulenchromatographie über Silicagel mit Tetrachlormethan/Acetonitril als mobile Phase gereinigt. Nach dem Abtrennen des Laufmittels erhielt man 9,9 g (0,03 mol) 2-(2,4-Dichlorbenzoyl)-2-(1,2,4-triazol-1-yl)-essigsäureethylester in Form eines Öls.

Beispiel 2:

In einem Reaktionskolben wurden 15,36 g (0,06 mol) 2-(2,4-Dichlorphenyl)-1-(1,2,4-triazol-1-yl)-ethan-2-on in 400 ml Methylenchlorid und 6,1 g - (0,06 mol) Triethylamin gelöst. Nach Kühlung auf 5°C wurden 7,11 g (0,06 mol) Chlorameisensäure-(2-propyl)-ester in 50 ml Methylenchlorid unter Konstanthaltung der Temperatur zugetropft. Nach beendeter Reaktion wurde die Reaktionslösung mit Wasser gewaschen, das Lösungsmittel abgedampft und der verbleibende Rückstand über Säulenchromatographie gereinigt. Es wurden 17 g - (0,05 mol) 2-(2,4-Dichlorbenzoyl)-2-(1,2,4-triazol-1-yl)-essigsäurepropylester mit Fp. 76 - 81°C erhalten.

Beispiel 3:

14,08 g (0,055 mol) 2-(2,4-Dichlorphenyl)-1-(1,2,4-triazol-1-yl)-ethan-2-on wurden in 100 g (0,98 mol) Triethylamin aufgeschlämmt und auf 10°C gekühlt. Anschließend wurden 5,2 g (0,055 mol) Chlorameisensäuremethylester zugetropft. Nach beendeter Reaktion wurde die Lösung eingeengt und der Rückstand in Diethylether aufgenommen, worauf der unlösliche Rückstand abfiltriert wurde. Nach dem Verdampfen des Lösungsmittels wurden 15,5 g (0,05 mol) 2-(2,4-Dichlorbenzoyl)-2-(1,2,4-triazol-1-yl)-essigsäuremethylester mit Fp. 105°C erhalten.

Beispiel 4:

Zu einer Lösung von 10,1 g (0,0295 mol) 2-(2,4-Dichlorbenzoyl)-2-(1,2,4-triazol-1-yl)-essigsäurepropylester in 100 ml Diisopropylether wurde bei einer Temperatur von 5°C unter Rühren eine HCl-gesättigte Diisopropyletherlösung so lange zugetropft, bis die Niederschlagsbildung beendet war. Der kristalline Niederschlag wurde unter Kühlung abfiltriert, mit Diisopropylether gewaschen und bei Raumtemperatur getrocknet. Es wurden 6,5 g (0,017 mol) 2-(2,4-Dichlorbenzoyl)-2-(1,2,4-triazol-1-yl)-essigsäurepropylester-Hydrochlorid mit Fp.105 -108 °C erhalten.

Beispiel 5:

8,42 g (0,025 mol) 2-(2,4-Dichlorbenzoyl)-2-(1,2,4-triazol-1-yl)-essigsäure-n-propylester wurden in 40 ml Ethanol gelöst und unter Rühren bei Raumtemperatur 1,18 g (0,0123 mol) Zinkchlorid gelöst in 50 ml Ethanol zugetropft. Nach etwa einer Stunde wurde der erhaltene Niederschlag abfiltriert, mit Ethanol gewaschen und getrocknet. Es wurden 6,8 g (0,0082 mol) [2-(2,4-Dichlorbenzoyl)-2-(1,2,4-triazol-1-yl)-essigsäure-n-propylester]$_2$.$ZnCl_2$ mit Fp. 120 -125,5°C erhalten.

Auf einem der beschriebenen Wege wurden die in der Tabelle zusammengefaßten und durch ihre Schmelzpunkte charakterisierten Verbindungen erhalten (*.....Zersetzung).

| Nr. | $R_1$ | $R_2$ | Y | Z | Salz | Fp/°C/ |
|---|---|---|---|---|---|---|
| 6 | Cl | Cl | O | O-Cyclohexyl | - | Öl |
| 7 | Cl | Cl | O | O-4-butylcyclohexyl | - | Öl |
| 8 | Cl | Cl | O | O-phenyl | - | 122-126 |
| 9 | Cl | Cl | O | O-$C_6H_5COOCH_3$ | - | 106-115 |
| 10 | Cl | Cl | O | O-benzyl | - | Öl |
| 11 | Cl | Cl | O | O-2,4-dichlorbenzyl | - | 100-104 |
| 12 | Cl | Cl | O | O-propyl | 1/2 $CuCl_2$ | 135-150 |
| 13 | Cl | Cl | O | O-propyl | 1/2 $SnCl_2$ | 156-162.5 |
| 14 | Cl | Cl | O | O-1,1,1-trichlorethyl | - | Öl |
| 15 | Cl | Cl | O | O-hexyl | - | 55-56 |
| 16 | Cl | Cl | O | O-octyl | - | Öl |
| 17 | Cl | Cl | O | O-decyl | - | Öl |
| 18 | Cl | Cl | O | O-octadecyl | - | Öl |
| 19 | Cl | Cl | O | O-i-butyl | - | 57-60 |
| 20 | Cl | Cl | O | O-trichlor-i-butyl | - | Öl |
| 21 | Cl | Cl | O | O-methoxyethyl | - | Öl |
| 22 | Cl | Cl | O | O-allyl | - | Öl |
| 23 | Cl | Cl | O | O-vinyl | - | Öl |
| 24 | Cl | Cl | O | O-methoxycarbonylpropyl | - | Öl |
| 25 | Cl | Cl | O | O-i-propyl | - | 60°C |
| 26 | Cl | Cl | O | O-butyl | - | Öl |
| 27 | Cl | Cl | O | O-1-methylbutyl | - | Öl |
| 28 | Cl | Cl | O | O-sec.butyl | - | Öl |
| 29 | Cl | Cl | O | O-2-methylpentyl | - | Öl |
| 30 | Cl | Cl | O | O-2-butinyl | - | 73-75 |
| 31 | Cl | Cl | O | O-1-butinyl | - | 60 |

| | | | | | | |
|---|---|---|---|---|---|---|
| 32 | Cl | Cl | O | O-propinyl-1 | - | 76 - 81 |
| 33 | Cl | Cl | O | S-ethyl | - | 59 - 60 |
| 34 | Cl | Cl | O | S-i-propyl | - | 86 - 89 |
| 35 | Cl | Cl | O | S-sec.-butyl | - | Öl |
| 36 | Cl | Cl | O | S-heptyl | - | Öl |
| 37 | Cl | Cl | O | S-octyl | - | Öl |
| 38 | Cl | Cl | O | S-dodecyl | - | 37 - 38 |
| 39 | Cl | Cl | S | O-benzyl | - | 174 - 182* |
| 40 | Cl | Cl | O | dimethylamino | - | Öl |
| 41 | Cl | Cl | O | diethylamino | - | Öl |
| 42 | Cl | Cl | O | diphenylamino | - | 162 - 166 |
| 43 | Cl | Cl | O | methyl-ethoxycarbonylamino | - | 83 - 88 |
| 44 | Cl | Cl | O | 3,5-dimethylmorpholino | - | 124 - 126 |
| 45 | Br | H | O | O-ethyl | - | 121 - 124 |
| 46 | F | H | O | O-ethyl | - | 87 - 90 |
| 47 | Methoxy | H | O | O-ethyl | - | 97 - 102 |
| 48 | Cl | Cl | O | O-brompropyl | - | 72 - 82 |
| 49 | Cl | Cl | O | O-trimethylsilylpropyl | - | Öl |

Beispiel 50:

1 Teil 2-(2,4-Dichlorbenzoyl)-2-(1,2,4-triazol-1-yl)-essigsäureethylester wurde in 3 Teilen Xylol gelöst und mit einem Teil einer Mischung aus 50 % Cadodeclybenzolsulfonat und 50 % ethoxyliertem Nonylphenol mit einem durchschnittlichen Gehalt von 10 Molen Ethylenoxid/1 Mol Nonylphenol versetzt. Dieses Konzentrat, enthaltend 20 Gew.% Wirkstoff, konnte mit Wasser zu einer Emulsion der gewünschten Konzentration verdünnt werden.

Beispiel 51:

1 Teil 2-(2,4-Dichlorbenzoyl)-2-(1,2,4-triazol-1-yl)-essigsäureethylester wurden mit 2 Teilen Attaclay, 6 Teilen Kaolin und 1 Teil Na-oleylmethyltaurid vermengt und in einer Planetenkugelmühle 1 Stunde gemahlen. Das so erhaltene benetzbare Pulver konnte in Wasser zu einer Spritzbrühe der gewünschten Konzentration dispergiert werden.

Beispiel 52:

1 Teil 2-(2,4-Dichlorbenzoyl)-2-(1,2,4-triazol-1-yl)-essigsäure-n-propylester wurde in 3 Teilen Xylol gelöst und mit 1 Teil einer Mischung aus 40 % Cadodecylbenzolsulfonat und 60 % eines Polyoxyethylen-sorbitan-fettsäureesters versetzt. Durch Vermischen mit Wasser konnte eine Emulsion der gewünschten Konzentration erhalten werden.

Beispiel 53:

1 Teil 2-(2,4-Dichlorbenzoyl)-2-(1,2,4-triazol-1-yl)-essigsäure-n-propylester wurde mit 2 Teilen hochdisperser gefällter Kieselsäure, 6 Teilen Kaolin und 1 Teil einer Mischung aus 30 % Dialkylnaphthalinsulfonsäure-Na-salz und 70 % Naoleylmethyltaurid vermengt und in einer Planetenkugelmühle 1 Stunde lang vermahlen. Das so erhaltene benetzbare Pulver wurde in Wasser zu einer Spritzbrühe der gewünschten Konzentration verdünnt.

Beispiel A:

Prophylaktische Wirkung der Verbindungen gegen echten Mehltau (Erysiphe cichoracearum) auf Gurken durch Behandlung der Blätter

10 Tage alte Gurkenpflanzen wurden mit einer wäßrigen Verdünnung des formulierten Wirkstoffes besprüht. Nach dem Trocknen der Spritzflüssigkeit wurden die Pflanzen mit von infizierten Pflanzen gewonnenen Sporen inokuliert. Die Gurkenpflanzen blieben anschließend im Gewächshaus bei rund 60 % Luftfeuchtigkeit stehen.

14 Tage nach der künstlichen Infektion wurde der Befall mit Erysiphe cichoracearum nach folgendem Boniturschema ausgewertet:

1 = kein Befall

2 = bis 2,5 % der Blattfläche befallen

3 = bis 5,0 % der Blattfläche befallen

4 = 6 -10 % der Blattfläche befallen

5 = 11 -15 % der Blattfläche befallen

6 = 16 - 25 % der Blattfläche befallen

7 = 26 -35 % der Blattfläche befallen

8 = 36 -67 % der Blattfläche befallen

9 = mehr als 67 % der Blattfläche befallen

Die Wirkstoffe, die Wirkstoffkonzentrationen und die Boniturwerte sind aus der nachfolgenden Tabelle ersichtlich.

| Wirkstoff | Wirkstoffkonzentrationen (Gew.%) 0,1 % | 0,05 % | 0,01 % | 0,005 % | 0,001 | 0,0005% |
|---|---|---|---|---|---|---|
| nach Beispiel 3 | 1 | 1 | 1 | 1 | 3 | - |
| nach Beispiel 1 | 1 | 1 | 1 | 1 | 1 | 2 |
| 20 | 1 | 1 | 1 | 1 | 5 | 7 |
| 29 | 1 | 1 | 1 | 1 | 2 | - |
| 38 | 1 | 1 | 1 | 1 | 2 | 4 |
| 25 | 1 | 1 | 1 | 1 | - | - |
| 28 | 1 | 1 | 1 | 1 | 7 | - |
| 31 | 1 | 1 | 1 | 1 | 5 | - |
| 6 | 1 | 1 | 1 | 1 | 7 | - |
| 16 | 1 | 1 | 1 | 1 | 5 | - |
| 37 | 1 | 1 | 1 | 1 | 4 | - |

Beispiel B:

Prophylaktische Wirkung der Verbindungen gegen Getreidemehltau (Erysiphe graminis) auf Weizen durch Behandlung der Blätter

Etwa 10 cm große Weizenpflanzen wurden mit einer wäßrigen Verdünnung des formulierten Wirkstoffes allseits besprüht. Nach 48 Stunden wurden die behandelten Pflanzen mit von infizierten Pflanzen gewonnenen Conidiosporen des Pilzes Erysiphe graminis var. tritici bestäubt.

Bei rund 22 Grad Celsius blieben die infizierten Pflanzen im Gewächshaus stehen. 10 Tage nach der künstlichen Infektion wurde ausgewertet.

Dabei verhinderten die Verbindungen nach Beispiel 1, nach Beispiel 2, 22, 26, 40, 24, 36, 8, 38, 34 und 35 das Wachstum des Pilzes vollständig.

Beispiel C:

Prophylaktische Wirkung der Verbindungen gegen echten Mehltau (Podosphaera leucotricha) an Apfelsämlingen

Junge Apfelsämlinge wurden mit einer wäßrigen Verdünnung des formulierten Wirkstoffes allseits besprüht und blieben anschließend für 24 Stunden im Gewächshaus stehen.

Mit von erkrankten Apfelsämlingen gewonnenen Conidiosporen wurden die behandelten Pflanzen anschließend inokuliert.

Nach Ausbruch der Krankheit an den Kontrollpflanzen wurde der Befall mit Podosphaera leucotricha bewertet.

Dabei verhinderten beispielsweise die Verbindungen nach Beispiel 1 und nach Beispiel 2 in einer Wirkstoffkonzentration von 0,005 % das Wachstum des Pilzes vollständig, bei den Verbindungen 33 und 24 waren rund 3 % der Blattfläche befallen.

Beispiel D:

Prophylaktische Wirkung gegen Weizenbraunrost - (Puccinia recondita) an Weizen

Zur Prüfung auf protektive Wirksamkeit wurden junge Weizenpflanzen mit einer wäßrigen Sporensuspension von Puccinia recondita inokuliert.

Nach dem Antrocknen besprüht man die Pflanzen mit der Wirkstoffzubereitung bis zur Tropfnässe. Anschließend wurden die Pflanzen für 24 Stunden bei 20°C und rund 95 % Luftfeuchtigkeit in einem Klimaschrank inokuliert. Bis zum Ausbruch der Krankeit an den Kontrollpflanzen standen die Testpflanzen im Gewächshaus bei rund 20°C und 70 % Luftfeuchtigkeit.

Wirkstoff, Wirkstoffkonzentrationen und Boniturwerte (Boniturschema 1 -9 aus Beispiel 1) sind aus der nachfolgenden Tabelle ersichtlich.

| Wirkstoffe | Wirkstoffkonzentrationen (%) 0,01 | 0,005 | 0,0025 |
|---|---|---|---|
| nach Beispiel 2 | 1 | 2 | 2 |
| 22 | 1 | 2 | 2 |
| 26 | 1 | 2 | 4 |
| 8 | 1 | 2 | 5 |
| 24 | 1 | 2 | 2 |
| nach Beispiel 5 | 1 | 2 | - |

Beispiel E:

Fungizide Wirklsamkeit im Agar-Plattentest

Die in Aceton gelösten Wirkstoffe wurden in bestimmter Konzentration einem verflüssigten Nährboden zugesetzt.

Der Agar wurde in Petrischalen ausgegossen und nach dem Erkalten mit dem Testorganismus beimpft. Sobald die Kontrolle vom Testorganismus bewachsen war, wurde ausgewertet.

In der nachfolgenden Tabelle ist als minimale Hemmkonzentration (MHK) die geringste im Nährboden enthaltene Konzentration (in Gew.%) der Substanz angegeben, bei der das Pilzwachstum völlig gehemmt war.

| Wirkstoff | Rhizoctonia Solani | Poria Placenta | Merulius Lacrimans | Pseudocercosporella herpotrichoides |
|---|---|---|---|---|
| nach Beispiel 3 | 0,04 | 0,04 | 0,008 | - |
| 22 | 0,04 | 0,04 | 0,0016 | 0,04 |
| 26 | 0,008 | - | 0,008 | - |
| 29 | 0,0016 | - | 0,0016 | - |
| 24 | 0,008 | 0,04 | 0,008 | - |
| 34 | - | 0,04 | 0,04 | - |
| nach Beispiel 1 | - | - | - | 0,008 |

Beispiel F:

Fungizide und bakterizide Wirksamkeit im Agar-Diffusionstest

Von den Wirkstofformulierungen wurden wäßrige Dispersionen hergestellt. Filterpapierplättchen wurden darin getränkt und anschließend auf den beimpften Nährboden gelegt.

Sobald die Kontrolle vom Testorganismus bewachsen war, wurde ausgewertet.

Es verhinderte beispielsweise die Verbindung 29 in einer Wirkstoffkonzentration von 0,0016 % das Wachstum von Alternaria tenuis vollständig, in einer Konzentration von 0,008 % 35 und 34. Mit 0,04 % Wirkstoffkonzentration wurde von den Verbindungen 29, 22, 26, 28, 24 und der Verbindung nach Beispiel 2 das Wachstum von Penicillium glaucum total blockiert.

Das Wachstum des Bakteriums Xanthomonas ssp. wurde beispeilsweise bei einer Wirkstoffkonzentration von 0,04 % von der Verbindung 35 vollständig verhindert.

Beispiel G:

Einfluß der Wirkstoffe auf das Wachstum der Alge Chlorella fusca

Zu 70 ml Algensuspension, entsprechend 0,1 mg Algen/1 ml Nährlösung, wurde die zu prüfende Substanz, gelöst in Aceton, zugegeben.

Die Algensuspensionen wurden 48 Stunden lang bei künstlicher Beleuchtung und Belüftung gehalten.

Ausgewertet wird der Prozentwert (P) = Algenkonzentration (in mg) in der Testlösung im Verhältnis zur Kontrolle (= 100 %).

In einer Wirkstoffkonzentration von 10 ppm war beispielsweise bei der Verbindung nach Beispiel 2 P kleiner als 1 %.

**Ansprüche**

1. Benzoyl-triazolyl-essigsäure-oder thioessigsäurederivate der allgemeinen Formel I des Formelblattes,

in der $R_1$ und $R_2$ unabhängig voneinander Wasserstoff, Halogen oder einen Alkoxyrest mit 1 -4 C-Atomen, Y Sauerstoff oder Schwefel, Z die Reste $OR_4$, $SR_4$ oder $R_5NR_6$, wobei $R_4$ einen geradkettigen oder verzweigten, gesättigten oder ungesättigten, unsubstituierten oder ein-oder mehrfach durch Halogen-, Alkoxy-oder Alkoxycarbonyl mit 1 bis 4 C-Atomen in der Alkylkette, Phenyl-, Halogenphenyl-oder Trialkylsilylreste mit 1 -4 C-Atomen in der Alkylkette substituierten Kohlenwasserstoffrest mit 1 bis 20 C-Atomen oder einen cyclischen Kohlenwasserstoffrest, der durch Alkyl-oder Alkoxycarbonylreste mit 1 -4 C-Atomen in den Alkylketten substituiert sein kann und $R_5$ und $R_6$ unabhängig voneinander einen Alkyl-, einen Alkoxycarbonylrest mit 1 -4 C-Atomen in den Alkylketten, einen Phenylrest oder $R_5$ und $R_6$ gemeinsam mit dem Stickstoffatom einen Morpholinoring, der durch Alkylreste mit 1 -4 C-Atomen substituiert sein kann, bedeuten und deren pflanzenphysiologisch verträgliche Salze und Metallkomplexe.

2. Benzoyl-triazolyl-essigsäure-oder -thioessigsäurederivate gemäß Anspruch 1 der allgemeinen Formel I, in der $R_1$ und $R_2$ unabhängig voneinander ein Halogenatom oder den Methoxyrest, Y Sauerstoff oder Schwefel, Z die Reste $OR_4$, $SR_4$ oder $R_5NR_6$, wobei $R_4$ einen geradkettigen oder verzweigten, gesättigten oder ungesättigten, unsubstituierten oder ein-oder mehrfach durch Halogenatome, Alkoxyreste mit 1 -4 C-Atomen oder

halogenierte Phenylreste substituierten Kohlenwasserstoffrest mit 1 -18 C-Atomen oder einen Cyclohexylrest und $R_5$ und $R_6$ je einen Alkylrest mit 1 -4 C-Atomen bedeuten und deren pflanzenphysiologisch verträgliche Salze und Metallkomplexe.

3. Benzoyl-triazolyl-essigsäure-oder thioessigsäurederivate gemäß Anspruch 1 der allgemeinen Formel I, in der $R_1$ und $R_2$ je ein Chloratom, Y Sauerstoff, Z die Reste $OR_4$, $SR_4$, wobei $R_4$ einen unsubstituierten oder ein-oder mehrfach durch Chlor substituierten Alkylrest mit 1 -15 C-Atomen, den Benzylrest oder den 2,4-Dichlorbenzylrest bedeuten und deren pflanzenphysiologisch verträgliche Salze und Metallkomplexe.

4. Verfahren zur Herstellung der Verbindungen der allgemeinen Formel I, dadurch gekennzeichnet, daß man ein Keton der allgemeinen Formel II des Formelblattes,

in der $R_1$ und $R_2$ die in Anspruch 1 angegebene Bedeutung haben, mit einer Verbindung der allgemeinen Formel III des Formelblattes,

in der Y und Z die in Anspruch 1 angegebene Bedeutung haben und Hal Chlor oder Brom bedeutet, in Gegenwart der mindestens äquivalenten Menge eines Säureakzeptors und gegebenenfalls in Gegenwart eines unter Reaktionsbedingungen inerten Verdünnungsmittels bei einer Temperatur von -20°C bis 140°C umsetzt, gegebenenfalls das Verdünnungsmittel entfernt und das Reaktionsprodukt gegebenenfalls in ein pflanzenphysiologisch verträgliches Salz oder Metallkomplex überführt.

5. Verfahren nach Anspruch 4, dadurch gekennzeichnet, daß man zuerst eine Verbindung der allgemeinen Formel II mit einem Säureakzeptor und anschließend mit einer Verbindung der allgemeinen Formel III umsetzt.

6. Verfahren nach Anspruch 4, dadurch gekennzeichnet, daß als Säureakzeptor Natriumhydrid oder ein tertiäres Amin eingesetzt wird.

7. Biozides Mittel, gekennzeichnet durch den Gehalt an mindestens einer Verbindung der allgemeinen Formel I des Formelblattes,

in der $R_1$ und $R_2$ unabhängig voneinander Wasserstoff, Halogen oder einen Alkoxyrest mit 1 -4 C-Atomen, Y Sauerstoff oder Schwefel, Z die Reste $OR_4$, $SR_4$ oder $R_5NR_6$, wobei $R_4$ einen geradkettigen oder verzweigten, gesättigten oder ungesättigten, unsubstituierten oder ein-oder mehrfach durch Halogen-, Alkoxy-oder Alkoxycarbonyl mit 1 bis 4 C-Atomen in den Alkylketten, Phenyl-, Halogenphenyl-oder Trialkylsilylreste mit 1 -4 C-Atomen in der Alkylkette substituierten Kohlenwasserstoffrest mit 1 bis 20 C-Atomen oder einen cyclischen Kohlenwasserstoffrest, der durch Alkyl-oder Alkoxycarbonylreste mit 1 -4 C-Atomen in den Alkylketten substituiert sein kann, und $R_5$ und $R_6$ unabhängig voneinander einen Alkyl-, einen Alkoxycarbonylrest mit 1 -4 C-Atomen in den Alkylketten, einen Phenylrest oder $R_5$ und $R_6$ gemeinsam mit dem Stickstoffatom einen Morpholinoring, der durch Alkylreste mit 1 -4 C-Atomen substituiert sein kann, bedeuten und deren pflanzenphysiologisch verträgliche Salze und Metallkomplexe.

8. Fungizides Mittel, gekennzeichnet durch den Gehalt an mindestens einer Verbindung der allgemeinen Formel I des Formelblattes,

in der $R_1$ und $R_2$ unabhängig voneinander Wasserstoff, Halogen oder einen Alkoxyrest mit 1 - 4 C-Atomen, Y Sauerstoff oder Schwefel, Z die Reste $OR_4$, $SR_4$ oder $R_5NR_6$, wobei $R_4$ einen geradkettigen oder verzweigten, gesättigten oder ungesättigten, unsubstituierten oder ein-oder mehrfach durch Halogen-, Alkoxy-oder Alkoxycarbonyl mit 1 bis 4 C-Atomen in den Alkylketten, Phenyl-, Halogenphenyl-oder Trialkylsilylreste mit 1 -4 C-Atomen in den Alkylketten substituierten Kohlenwasserstoffrest mit 1 bis 20 C-Atomen oder einen cyclischen Kohlenwasserstoffrest, der durch Alkyl-oder Alkoxycarbonylreste mit 1 -4 C-Atomen in den Alkylketten substituiert sein kann, und $R_5$ und $R_6$ unabhängig voneinander einen Alkyl-, einen Alkoxycarbonylrest mit 1 -4 C-Atomen in den Alkylketten, einen Phenylrest oder $R_5$ und $R_6$ gemeinsam mit dem Stickstoffatom einen Morpholinoring, der durch Alkylreste mit 1 -4 C-Atomen substituiert sein kann, bedeuten und deren pflanzenphysiologisch verträgliche Salze und Metallkomplexe.

9. Verfahren zur Bekämpfung von Schadorganismen, dadurch gekennzeichnet, daß man mindestens eine Verbindung der allgemeinen Formel I auf Schadorganismen und/oder deren Lebensraum einwirken läßt.

10. Verwendung von Verbindungen der allgemeinen Formel I zur Bekämpfung von Schadorganismen.

11. Verfahren zur Herstellung von bioziden Mitteln, dadurch gekennzeichnet, daß man Verbindungen der allgemeinen Formel I mit Streckmitteln und/oder oberflächenaktiven Mitteln vermischt.

Claims for contracting state AT

1. Biozides Mittel, gekennzeichnet durch den Gehalt an mindestens einer Verbindung der allgemeinen Formel I des Formelblattes,

in der $R_1$ und $R_2$ unabhängig voneinander Wasserstoff, Halogen oder einen Alkoxyrest mit 1 -4 C-Atomen, Y Sauerstoff oder Schwefel, Z die Reste $OR_4$, $SR_4$ oder $R_5NR_6$, wobei $R_4$ einen geradkettigen oder verzweigten, gesättigten oder ungesättigten, unsubstituierten oder ein-oder mehrfach durch Halogen-, Alkoxy-oder Alkoxycarbonyl mit 1 bis 4

C-Atomen in den Alkylkette, Phenyl-, Halogenphenyl-oder Trialkylsilylreste mit 1 -4 C-Atomen in der Alkylkette substituierten Kohlenwasserstoffrest mit 1 bis 20 C-Atomen oder einen cyclischen Kohlenwasserstoffrest, der durch Alkyl- oder Alkoxycarboanylreste mit 1 -4 C-Atomen in den Alkylketten substituiert sein kann und $R_5$ und $R_6$ unabhängig voneinander einen Alkyl-, einen Akoxycarbonylrest mit 1 -4 C-Atomen in den Alkylketten, einen Phenylrest oder $R_5$ und $R_6$ gemeinsam mit dem Stickstoffatom einen Morpholinoring, der durch Alkylreste mit 1 bis 4 C-Atomen substituiert sein kann, bedeuten und deren pflanzenphysiologisch verträgliche Salze und Metallkomplexe.

2. Biozides Mittel, gekennzeichnet durch den Gehalt an mindestens einer Verbindung der allgemeinen Formel I, in der $R_1$ und $R_2$ unabhängig voneinander ein Halogenatom oder den Methoxyrest, Y Sauerstoff oder Schwefel, Z die Reste $OR_4$, $SR_4$ oder $R_5NR_6$, wobei $R_4$ einen geradkettigen oder verzweigten, gesättigten oder ungesättigten, unsubstituierten oder ein-oder mehrfach durch Halogenatome, Alkoxyreste mit 1 -4 C-Atomen oder halogenierte Phenylreste substituierten Kohlenwasserstoffrest mit 1 -4 C-Atomen oder halogenierte Phenylreste substituierten Kohlenwasserstoffrest mit 1 -18 C-Atomen oder einen Cyclohexylrest und $R_5$ und $R_6$ je einen Alkylrest mit 1 -4 C-Atomen bedeuten und deren pflanzenphysiologisch verträgliche Salze und Metallkomplexe.

3. Biozides Mittel, gekennzeichnet durch den Gehalt an mindestens einer Verbindung der allgemeinen Formel I, in der $R_1$ und $R_2$ je ein Chloratom, Y Sauerstoff, Z die Reste $OR_4$, $SR_4$, wobei $R_4$ einen unsubstituierten oder ein-oder mehrfach durch Chlor substituierten Alkylrest mit 1 -15 C-Atomen, den Benzylrest oder den 2,4-Dichlorbenzylrest bedeuten und deren pflanzenphysiologisch verträgliche Salze und Metallkomplexe.

4. Fungizides Mittel, gekennzeichnet durch den Gehalt an mindestens einer Verbindung der allgemeinen Formel I des Formelblattes,

in der $R_1$ und $R_2$ unabhängig voneinander Wasserstoff, Halogen oder einen Alkoxyrest mit 1 -4 C-Atomen, Y Sauerstoff oder Schwefel, Z die Reste $OR_4$, $SR_4$ oder $R_5NR_6$, wobei $R_4$ einen geradkettigen oder verzweigten, gesättigten oder ungesättigten, unsubstituierten oder ein-oder mehrfach durch Halogen-, Alkoxy-oder Alkoxycarbonyl mit 1 -4 C-Atomen in den Alkylketten, Phenyl-, Halogenphenyl-oder Trialkylsilylreste mit 1 -4 CAtomen in den Alkylketten substituierten Kohlenwasserstoffrest mit 1 bis 20 C-Atomen oder einen cyclischen Kohlenwasserstoffrest, der durch Alkyl- oder Alkoxycarbonylreste mit 1 -4 C-Atomen in den Alkylketten substituiert sein kann, und $R_5$ und $R_6$ unabhängig voneinander einen Alkyl-, einen Alkoxycarbonylrest mit 1 -4 C-Atomen in den Alkylketten, einen Phenylrest oder $R_5$ und $R_6$ gemeinsam mit dem Stickstoffatom einen Morpholinoring, der durch Alkylreste mit 1 -4 C-Atomen substituiert sein kann, bedeuten und deren pflanzenphysiologisch verträgliche Salze und Metallkomplexe.

5. Verfahren zur Bekämpfung von Schadorganismen, dadurch gekennzeichnet, daß man mindestens eine Verbindung der allgemeinen Formel I auf Schadorganismen und=oder deren Lebensraum einwirken läßt.

6. Verwendung von Verbindungen der allgemeinen Formel I zur Be kämpfung von Schadorganismen.

7. Verfahren zur Herstellung von bioziden Mitteln, dadurch gekennzeichnet, daß man Verbindungen der allgemeinen Formel I mit Streckmitteln und/oder oberflächenaktiven Mitteln vermischt.

8. Verfahren zur Herstellung der Verbindungen der allgemeinen Formel I, dadurch gekennzeichnet, daß man ein Keton der allgemeinen Formel II des Formelblattes,

in der $R_1$ und $R_2$ die in Anspruch 1 angegebene Bedeutung haben, mit einer Verbindung der allgemeinen Formel III des Formelblattes,

in der Y und Z die in Anspruch 1 angegebene Bedeutung haben und Hal, Chlor oder Brom bedeutet, in Gegenwart der mindestens äquivalenten Menge eines Säureakzeptors und gegebenenfalls in Gegenwart eines unter Reaktionsbedingungen inerten Verdünnungsmittels bei einer Temperatur von -20°C bis 140°C umsetzt, gegebenenfalls das Verdünnungsmittel entfernt und das Reaktionsprodukt gegebenenfalls in ein pflanzenphysiologisch verträgliches Salz oder Metallkomplex überführt.

9. Verfahren nach Anspruch 8, dadurch gekennzeichnet, daß man zuerst eine Verbindung der allgemeinen Formel II mit einem Säureakzeptor und anschließend mit einer Verbindung der allgemeinen Formel III umsetzt.

10. Verfahren nach Anspruch 8, dadurch gekennzeichnet, daß als Säureakzeptor Natriumhydrid oder ein tertiäres Amin eingesetzt wird.

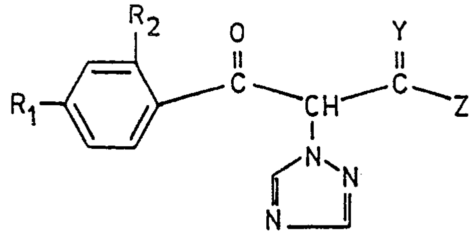

I

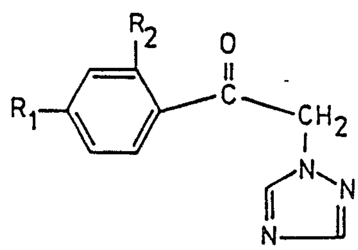

II

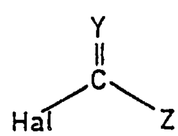

III

Europäisches Patentamt

# EUROPÄISCHER RECHERCHENBERICHT

Nummer der Anmeldung

EP 86108466.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CHEMICAL ABSTRACTS, Band 97, Nr. 25, 20. Dezember 1982, Columbus, Ohio, USA<br>SUMITOMO CHEMICAL CO., LTD. "Triazolyl ß-diketones".<br>Seite 855, Spalte 2, Zusammenfassung-Nr. 216 196u<br>& Jpn. Kokai Tokkyo Koho JP 82,140, 772<br>-- | 1 | C 07 D 249/08<br>A 01 N 43/64 |
| A | CHEMICAL ABSTRACTS, Band 97, Nr. 25, 20. Dezember 1982, Columbus, Ohio, USA<br>SUMITOMO CHEMICAL CO., LTD. "Triazylyl ketone derivatives".<br>Seite 856, Spalte 1, Zusammenfassung-Nr. 216 197v<br>& Jpn. Kokai Tokkyo Koho JP 82,140, 773<br>---- | 1 | RECHERCHIERTE SACHGEBIETE (Int. Cl.4)<br>C 07 D 249/00 |

Der vorliegende Recherchenbericht wurde für alle Patentansprüche erstellt.

| Recherchenort | Abschlußdatum der Recherche | Prüfer |
|---|---|---|
| WIEN | 02-10-1986 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82